Europäisches Patentamt

European Patent Office

Office européen des brevets

(11) Publication number: 0 358 429
A2

# (12) EUROPEAN PATENT APPLICATION

(21) Application number: 89308933.4

(22) Date of filing: 04.09.89

(51) Int. Cl.⁵: A 61 M 1/00

(30) Priority: 06.09.88 US 240992

(43) Date of publication of application:
14.03.90 Bulletin 90/11

(84) Designated Contracting States: DE FR GB

(71) Applicant: BAXTER INTERNATIONAL INC.
One Baxter Parkway
Deerfield, IL 60015 (US)

(72) Inventor: Borsanyi, Alexander
1191 Yacht Puritan
Newport Beach, CA 92660 (US)

Sorensen, John T.
2402 Harbor Boulevard
207 Costa Mesa, CA 92626 (US)

Lebo, Robert
28762 Sycamore Drive
Silverado, CA 92676 (US)

Stone, Albert L.
10 Tierra Vista
Laguna Vista, CA 92653 (US)

Harp, Richard
2122 Subida Terrace
La Costa, CA 92024 (US)

Pool, Scott L.
510 West Bell
Sana Ana, CA 92707 (US)

Rondinone, Joseph
27461 Almendra
Mision Viejo, CA 92691 (US)

Wall, Roxanne
P.O. Box 15296
Newport, CA 92659 (US)

(74) Representative: MacGregor, Gordon et al
ERIC POTTER & CLARKSON 14 Oxford Street
Nottingham, NG1 5BP (GB)

(54) Multimodal displacement pump and dissolution system for same.

(57) Disclosed is a pneumatically-driven bellows pump apparatus for oscillating liquid into a localized area of the human body, preferably methyl-tertiary butyl ether or another solvent, into the gallbladder. The pump includes a fluid reservoir in the form of an inverted syringe attached via a stopcock to the bellows. The bellows is attached via a second stopcock to a catheter also attached to a priming syringe. An inlet pinch valve is disposed between the bellows and the fluid reservoir and an outlet pinch valve between the bellows and the fluid receiver.

To use the pump, a pigtail catheter with outlets in the pigtail is inserted into the desired area of the body, preferably a receptacle such as the gallbladder, to introduce liquid for gallstone dissolution. Liquid is infused into the receptacle by decompression of the bellows with the inlet but not the outlet valve open and then by compression of the bellows with the outlet but not the inlet valve open. Repeated infusion and aspiration is accomplished by decompression and compression of the bellows with the inlet valve closed and the outlet valve open, or by compression and decompression of the bellows while the inlet valve is open and the outlet valve is closed alternated with compression and decompression while the inlet valve is closed and the outlet valve is open. Complete aspiration is accomplished by the use of a vacuum pump attached to the fluid reservoir. Also disclosed are syringe units and a transfer cap for use with the pump, together with a method for dissolving gallstones.

FIG. 1

Description

# MULTIMODAL DISPLACEMENT PUMP AND DISSOLUTION SYSTEM FOR SAME

## BACKROUND OF THE INVENTION

### Field of the Invention

This invention relates to pumps, particularly pumps for introducing fluids into the human body, as well as syringes, bottle caps and the like for use in connection with such pumps for gallstone dissolution and the like.

### Description of the Prior Art

Solidified masses such as biliary duct stones and gallstones may develop in hollow organs or ducts within humans and animals and cause numerous health problems, as is known to those skilled in the art. These deposits may be removed from the body in various ways, including surgery or in vivo dissolution by solvents introduced into the localized area of the body, such as the gallbladder, where the stones are located.

In order to effectively dissolve stones such as gallstones in vivo, however, an effective means of introducing and distributing the fluid is necessary; agitation of the fluid is advantageous and speeds up the dissolution process substantially; it can be accomplished by repeatedly cycling or oscillating the fluid into and out from the body area of interest. Furthermore, an effective dissolving agent is necessary; monooctanoin, for example, can be used, and methyl tertiary-butyl ether ("MTBE"), for example, has been found to be very effective, but is quite flammable. Any apparatus for injecting and aspirating MTBE requires the inclusion of safety precautions and the use of materials which are not degraded or dissolved by the solvent.

Pumps have been designed for use in such dissolution systems. For example, in U.S. patent 4,723,941, a piston pump is disclosed for oscillating methyl tertiary-butyl ether ("MTBE") and other solvents into the gallbladder. In U.S. patent No. 4,655,744, another pump is disclosed which provides a fluid trap between the gallbladder and the pump to remove the bile. However, an effective flexible but simple closed system pump with minimal air leakage and with optimum handling of all functions desireable in a dissolution pump has not previously been developed.

Other pumps having multimodal operation have been designed for various applications, such as that described in U.S. Patent No. 3,382,811, but require several displacement mechanisms and are designed to provide a constant output flow in the mainstream output line.

## SUMMARY OF THE INVENTION

The present invention includes infusion apparatus and related accessories particularly suited for gallstone dissolution, although useful for other purposes as well. The preferred embodiment is a pump apparatus which provides multiple modes of operation (i.e. infusion, aspiration, oscillation of more than one sort, and the like) useful for in vivo gallstone dissolution. In the preferred embodiment, air leakage into the pump system and the gallbladder is eliminated by the design of the unit, and control of the amount of solvent in the gallbladder is maintained. Furthermore, the preferred control system, a pneumatic logic module operating the unit minimizes any fire hazard associated with the use of electricity.

The invention in one aspect is an apparatus for transferring fluid having a fluid source, a transfer means for transferring fluid to or from the fluid source and a fluid receiver, a first means for closing off fluid flow from the source to the transfer means, a second means for closing off fluid flow from the transfer means to the receiver, and control means for independently controlling the transfer means and the first and the second closing means so that fluid can be transferred from the fluid source to the transfer means, from the transfer means to the fluid receiver, and back again, at the users selection.

In preferred embodiments, the control means functions so that fluid can also be repeatedly transferred directly from the transfer means to the fluid receiver and back again, and so that fluid can be repeatedly transferred directly from the fluid source to the transfer means and back again, at the user's selection.

In another aspect, the invention is a pump having a fluid source, means for transferring fluid to or from the fluid source and a fluid receiver, and pneumatic logic control means means to control the transfer of fluid from the fluid source to or from the fluid receiver.

Preferably, the transferring means is a positive displacement means such as a bellows, and the fluid source is an inverted graduated syringe so that fluid output and input can be measured. The first and second closing means are preferably pinch valves closed by pistons controlled by the logic. The bellows stroke is adjustable to control the volume of liquid it infuses and withdraws. Further, the bellows and the fluid source are both vertically aligned and designed to differentially transfer and retain the lower of any two phases of liquid they hold, in the fluid source. For the preferred use, bile is differentially transferred to the fluid source rather than retained in the bellows, and MTBE is infused.

As the apparatus is designed, the inlet and outlet valves are opened and closed so that the bellows, on extending and compressing, repeatedly withdraws and infuses fluid from and to either the fluid source or a fluid receiver (preferably the gallbladder reached via a catheter.). Alternately, the bellows and valves align so that the bellows withdraws fluid from the fluid source and injects it to the fluid receiver. Finally, all valves can be opened and the bellows compressed while fluid is withdrawn directly from the fluid receiver to the fluid source via a vacuum applied to the fluid source.

In another aspect, the invention includes a cap for a fluid container comprising a cap unit and tubing, the tubing disposed to extend through the cap unit into the container at one end and terminating in a luer seal at the other to facilitate withdrawal of liquid from the bottle by syringe. It also includes apparatus for withdrawing the plunger of a syringe with minimized air leakage including a syringe, a vacuum generator, and a vacuum chamber surrounding the syringe.

Finally, the invention in another aspect also includes a method for dissolving solidified masses in a localized area of the body such as the gallbladder by infusing a given volume of fluid into said localized area and oscillating a smaller volume to and from said localized area to dissolve said masses therein. Preferably, the solvent used is methyl tertiary-butyl ether, although other solvents such as monooctanoin can be used.

BRIEF DESCRIPTION OF THE DRAWINGS

Fig. 1 is a schematic diagram of the preferred pump system of the present invention.

Fig. 2 is a drawing of the preferred pump in use with a patient.

Figs 3 and 4 are cross sections of the preferred MTBE containers and transfer caps of the system of the present invention.

Figs 5 and 6 are side elevations of the preferred catheter to be used with the pump of the present invention.

Fig. 7 is a front elevation of the stopcock and threaded luer lock connecting the catheter and the pump of the present invention.

Fig. 8 is a front elevation of the piston of the main syringe of the preferred pump.

Figs 9, 10 and 11 are cross-sections of several embodiments of the bellows and transfer set of the preferred pump of the present invention.

Figs 12 and 13 are, respectively, a front elevation of the volume selection mechanism, the bellows, and the bellows drive piston and a side elevation of the bellows control mechanism in the preferred pump.

Fig. 14 is a cross-section of the preferred valve operating mechanism of the present invention.

Fig. 15 is a front elevation of the luer lock connectors of the present invention, connecting the preferred disposable transfer set to the rest of the preferred pump.

Figs 16, 17, and 18 are the left, middle, and right portions of the schematic covering the preferred pneumatic logic system operating the pump.

DESCRIPTION OF THE SPECIFIC EMBODIMENTS

The present pump 2 may have many uses, but is particularly effective for use in gallstone dissolution, where it pumps a fluid in and out of a fluid receptacle in the form of the gallbladder 4. For gallstone dissolution, the fluid is preferably methyl tertiary-butyl ether, although it can be another solvent such as monoctanoin or any subsequently discovered solvent or solvent system.

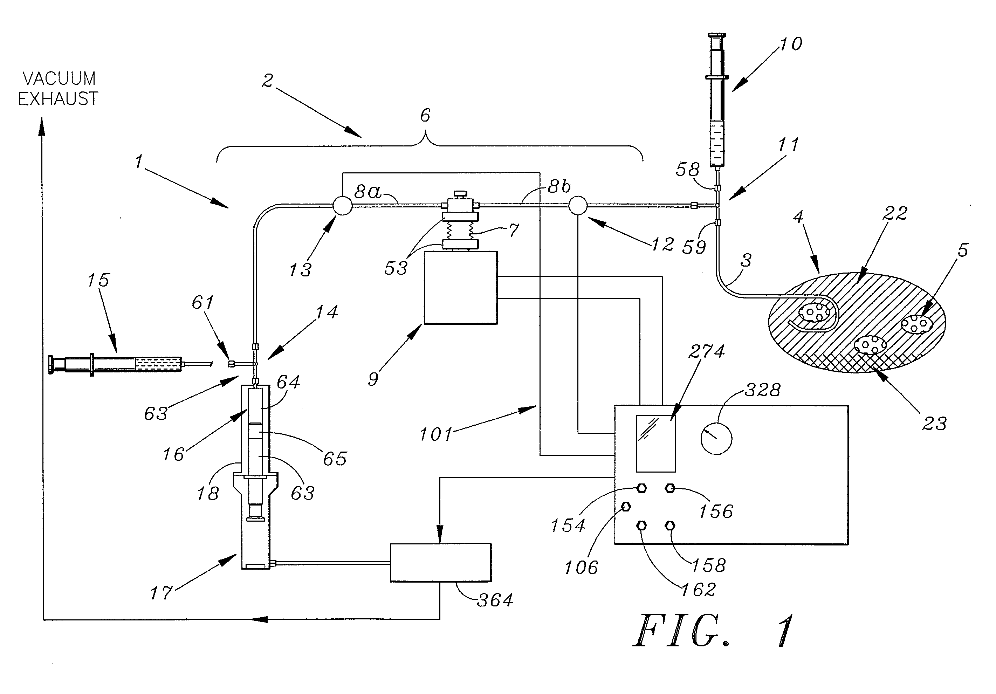

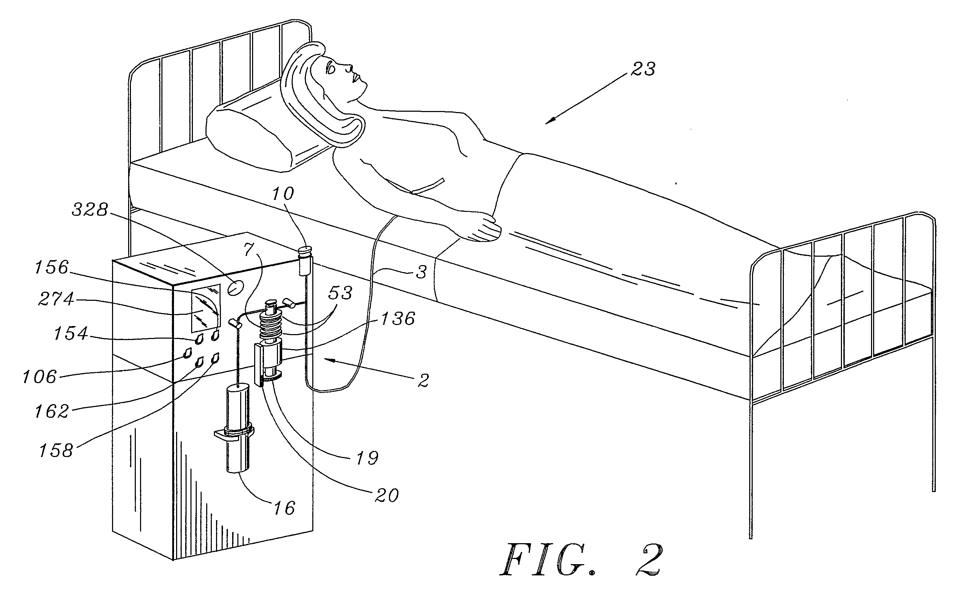

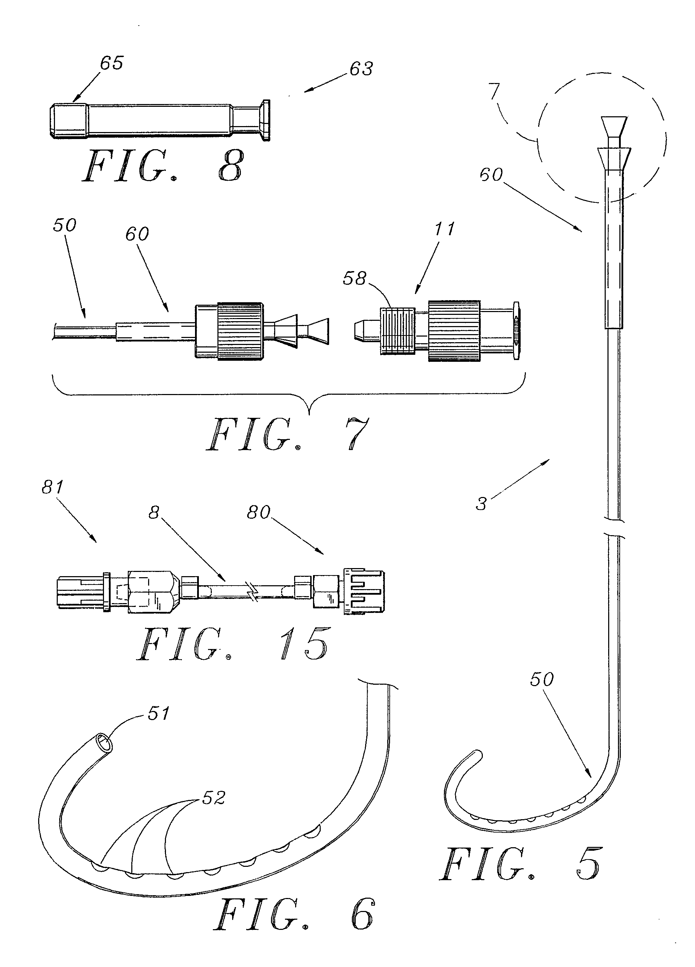

The system 1, as shown in Figs. 1 and 2, includes a single-lumen disposable catheter 3 which can be used independent of pump 1. It is shown in detail in Figs 5 and 6 and includes a pigtail 50 with an opening 51 at the far end plus fluid outlets 52 disposed along the inside length of the pigtail. When in use with the pump, the catheter 3 is attached via a threaded luer lock 59 (shown in Fig. 7) to stopcock 11 in the upper corner of the pump. The catheter includes a sheath 60 surrounding the pigtail at the upper end to prevent crimping or occlusion.

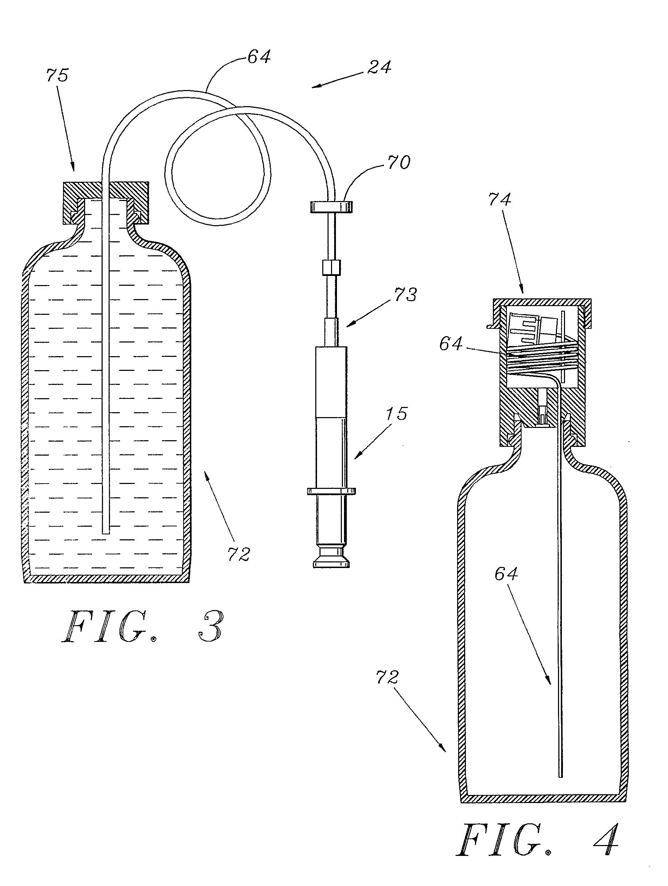

A transfer cap 24 shown in Fig. 3 is also provided as part of the system 1. It consists of a bottle cap 75 (for a bottle 72 of MTBE) having tubing 71 passing therethrough which terminates in a luer lock 73. Input syringes 15 for input of MTBE into the pump can be attached to the luer lock to be quickly filled with MTBE in a sterile fashion. The syringe can then be capped and the transfer cap closed off, for example by a slide clamp 70. As shown in Fig. 4, transfer cap 24 can include a removable, sealable cap 74 at the top of bottle cap 75 which contains helically wound tubing 64.

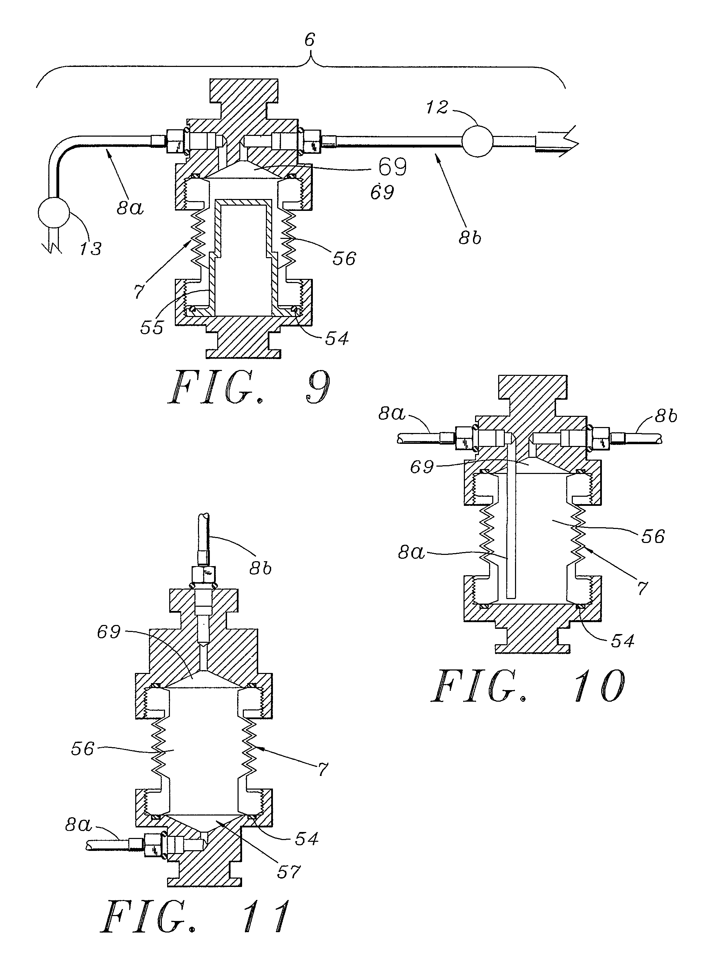

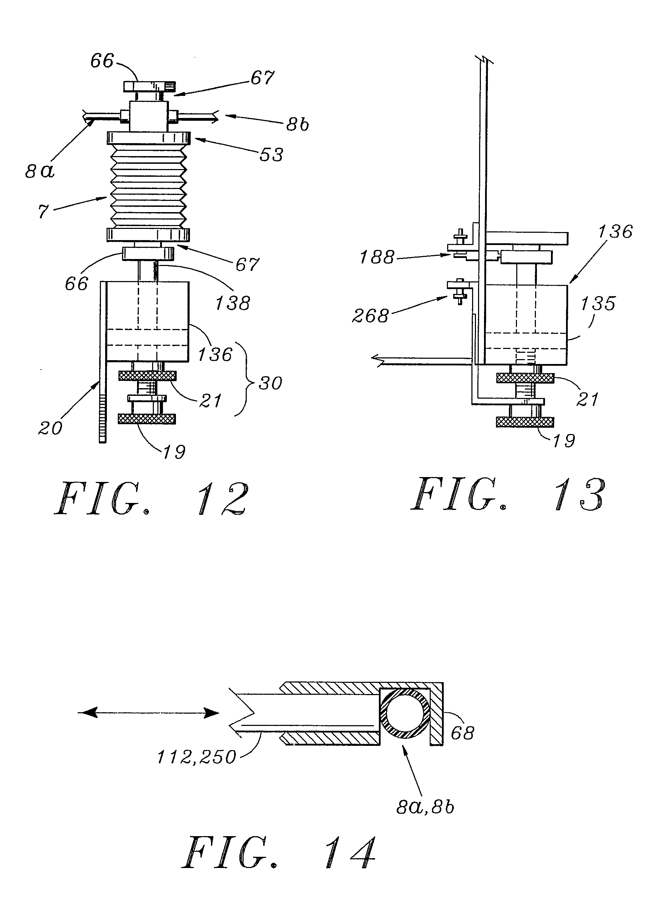

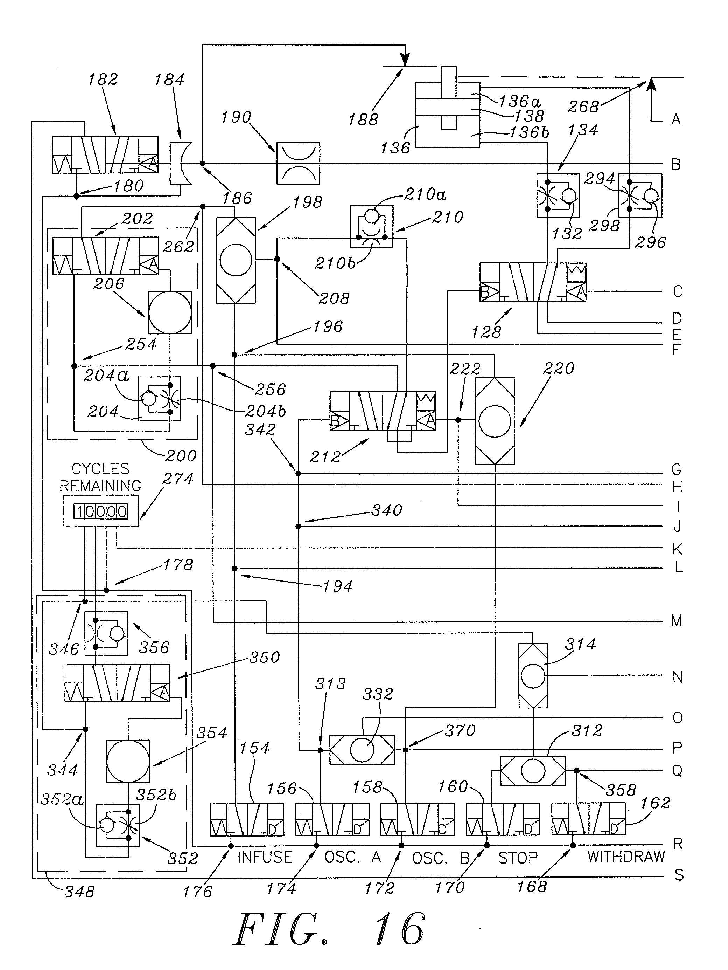

Returning to the pump 2, stopcock 11 is attached via another luer lock 58 to a standard 20 milliliter syringe 10 which is sealed off by the stopcock when the pump is in use. It is also attached to tubing 8b of disposable transfer set 6 which contains the displacement source (preferably bellows 7) of the pump by connector 81 shown in Fig. 15. This connector and similar connector 80 at the other end of the transfer set is a simple-to-use luer lock connector because the preferred transfer set is a disposable unit, replaced for each patient. Transfer set 6 is illustrated in Figs 1 and 12, and in more detail in Figs 9-11.

Transfer set 6 contains a flexible bellows 7 made of a flexible, compressible, material such as polypropylene. Other materials such as teflon, nylon, polyethylene cam be used, but must be essentially impervious to the fluid being pumped. A displacement source in the form of bellows is particularly desireable when the pump is used for gallstone dissolution because it provides a completely sealed system without the possibility of air leakage inherent in a piston unit, for example, and is very reliable.

The bellows terminates in top and bottom base 53 which extend to caps 66 via necks 67 which allow the unit to be suspended in place on the pump by hooks 85 not shown in the drawings. In the preferred embodiment, the bellows holds about 20 milliliters the bellows chamber 56 in its compressed position.

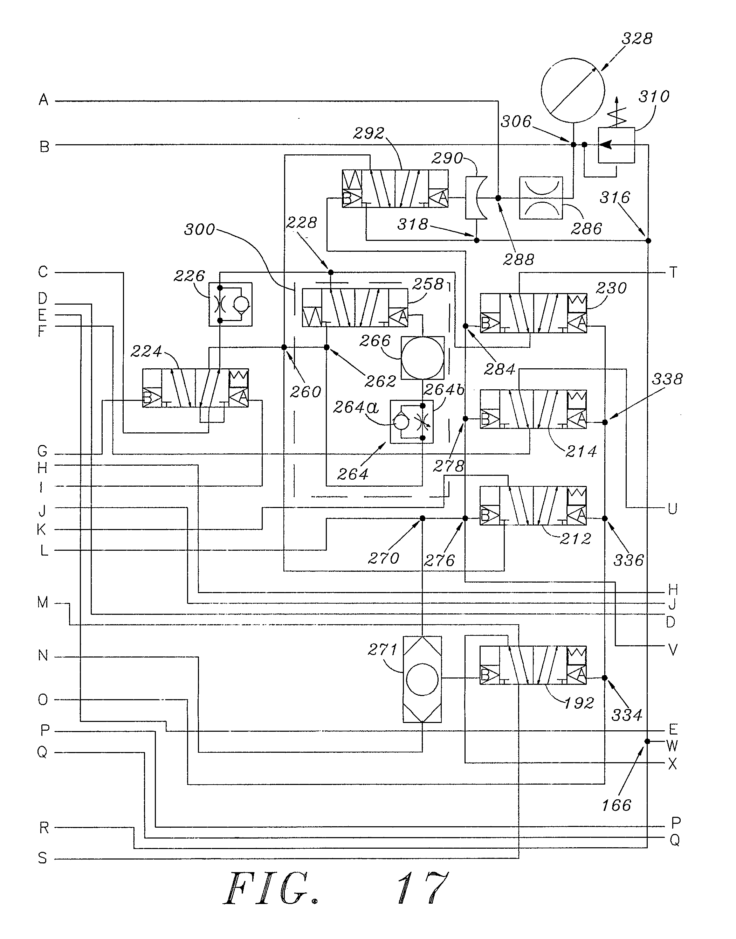

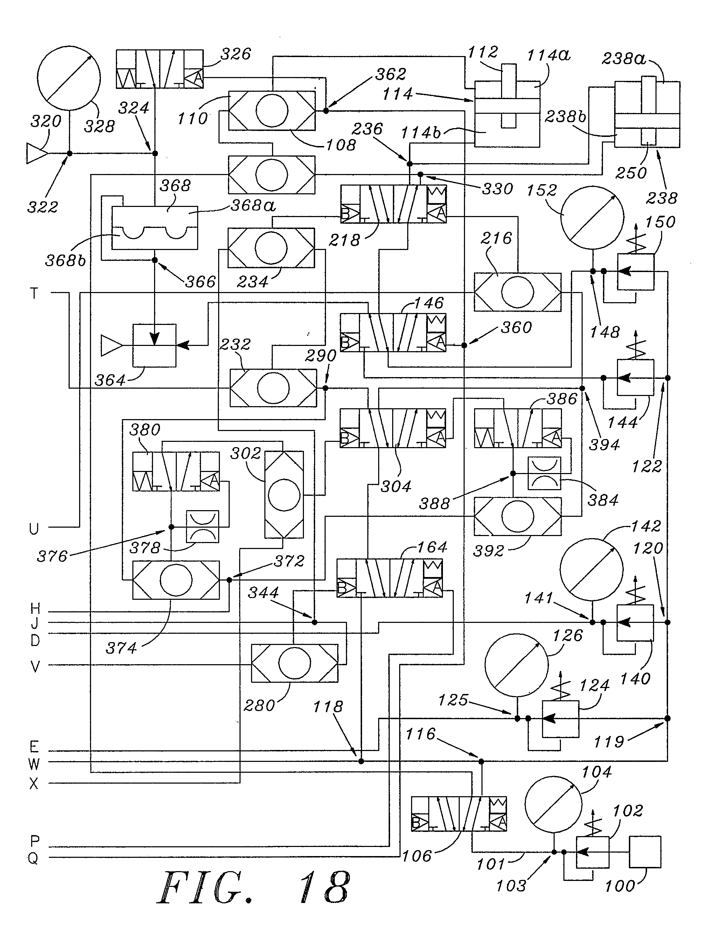

Tubing 8 extends from the transfer set 6 on each side to withdraw and add fluid to the bellows chamber 56, and is preferably made of nylon or another relatively MTBE-impervious polymer. Tubing 8, section 8b, passes through outlet valve 12, a pinch valve, while section 8a passes through inlet valve 13, also a pinch valve. As shown in Fig. 14, both valves have a surface post 68, so that tubing 8 can be compressed against it by a piston 112 or 250 (operated by the fluid logic of the pump as described later) to close the valve by closing off the tubing. It should be noted that in Figs. 16-19, the valve pistons 112 and 250 are shown schematically as moving "upward" and "downward," and are referred to as such. In actual operation, however, they are aligned and move horizontally in order to compress the tubing against post 68.

The bellows 7 are compressed when in "home" position; compression and extension are activated by a pneumatic logic system 9 positioned below the bellows and extending behind the pump; connecting tubing 101 is shown on Fig 1. A seal is created by o ring 54.

As shown in Figs 9, in one embodiment, the bellows includes a displacement cup 55 to absorb some of its volume; however, the bellows is preferably used without the displacement cup (particularly with oscillation A, not oscillation B, described later) because the larger fluid volume in bellows chamber 56 appears more desireable. (The MTBE used for dissolution is diluted more than in a smaller chamber and is thus more slowly saturated with dissolved cholesterol.)

Tubing 8a and 8b exit from the top of the bellows chamber 56 which is conical (69) at its upper end. In the embodiment shown in Fig. 10, tubing 8a extends adjacent the bottom of bellows chamber 56; as a result, when the bellows compresses, it differentially withdraws liquid at the lower portion of the chamber. Thus when MTBE (shown as 22 in gallbladder 4) and bile (shown as 23 in gallbladder 4) mix (the bile settling at the bottom and the MTBE remaining at the top), bile is differentially withdrawn from bellows chamber 56 by tubing 8a.

In the preferred embodiment shown in Fig. 11, bellows chamber 56 terminates at its lower end in a cone 57 and tubing 8a exits directly from the bottom also differentially withdrawing bile.

When transfer set 6 is attached to the pump 2, bellows 7 rests atop bellows drive piston 138 in bellows drive cylinder 136 as shown in Figs. 12, 13, and 16. The bottom of bellows drive piston 138 rests on the threaded end of knob 19 of volume selector 30. Knurled knob 19 threadably inserted into bellows drive cylinder 136 can be turned to adjust the position the bottom position of bellows drive piston 138 to control the extension of and the stroke volume of fluid delivered by the bellows. A gauge 20 extends from cylinder 136 to allow precise positioning of knob 19 to select the desired volume. Knob 21 locks knob 19 into position once set. Sensors 188 and 268, shown in Figs 13 and 16 sense when the bellows drive piston 138 reaches the top and bottom of its path and initiate the logic sequence to move it in the opposite direction when desired.

Tubing 8a is attached (via a connection as shown in Fig. 7) to stopcock 14 which in turn is attached via side luer lock 61 to input syringe 15, preferably a 20 milliliter standard syringe for injecting fluid into main syringe reservoir 16. Stopcock 14 seals off input syringe 15 when not in use, and attaches via a lower luer lock 63 to main syringe reservoir 16. Main syringe reservoir 16, a 20-25 milliliter syringe, contains a piston 63 which provides a seal with barrel 64 along only a portion, preferably along tip 65, of its length, to minimize a tendency of the piston 63 to become immobile due to crystallization of cholesterol in the MTBE where the piston seals.

Main reservoir 16 is held in sealed chamber 17 attached to vacuum pump 364 which exhausts into the hospital exhaust system, and which, when the vacuum is activated in the withdraw cycle of the pump, draws the piston 63 of reservoir 16 downward to withdraw liquid from the rest of the system into the reservoir. Because syringe 16 is volumetrically graduated, the exact amount of liquid going into and coming out of the system can be tracked and controlled.

The pump can be controlled by a number of different means known in the art, such as an electrical system, a microprocessor, a mechanical, or a hydraulic system. Software can be provided to program the sequence of events. In the preferred embodiment, fluid, preferably air or pneumatic logic is used as the control system because it eliminates any fire hazard from the use of electricity or the like where MTBE is used. Furthermore, most hospitals have an air supply in each room so that the use of pneumatic logic is convenient; even if there is no such air supply, air or nitrogen, being compressible, is convenient to keep and handle compared to liquid cylinders for hydraulic systems, for example, which would require large storage areas.

The entire preferred air logic system is shown in Figs. 16-19. A cylinder 100 of compressed air or nitrogen provides a pressure flow through the system. Alternately, an air compressor or other non-flammable gas source can be used. Air signals pass through the logic system to control the pump. The majority of the system is connected by tubing 101 of polyurethane, although other polymer or non-polymer materials can be used. Individual components of the system such as valves, OR gates, sensors, and the like are generally made of aluminum, stainless steel, or teflon in the preferred system.

Air from cylinder 100 passes through regulator 102 at a pressure controlled by the operator, passes by pressure gauge 104 which provides a readout of input pressure and from there into run/prime toggle valve 106 placed on the front of the pump. The run/prime valve is a detented toggle valve which can be purchased off the shelf. In essence, it is the power switch for the system.

This toggle valve, like all toggle valves, contains in essence two valves and toggles between them so that the user activates one or the other valve depending on the position of the toggle switch. Because this is a "detented" toggle valve, when the user flips the switch to one position or the other, the valve remains in the new position until re-toggled to the other position.

When toggled to "prime" or "off" position B, the air signal exits the run/prime valve through tubing system 101 to OR gate 108, OR gate 110, and inlet pinch valve control cylinder 114, upper segment 114a thereof, to force inlet pinch valve drive piston 112 downward to open inlet valve 13 as described via Fig. 14. This allows the entire system to be primed before use because both the inlet valve 13 and the outlet valve 12 (which remains in "home" position, i.e. open, as described later) are open, and the bellows is also in home.

Run/prime valve 106 when toggled to the "run" position activates portion A of the valve and is essentially a power switch. In run position, the air signal passes through the system 101 as follows. It passes through junction 116 and then in turn through junctions 118, 120, and 122. From junction 118, the signal passes through regulator 124 (set by the user) and pressure gauge 126 to provide a constant input signal at a first input channel to the bellows home valve 128, a detented pressure-piloted valve which can be purchased off the shelf. From junction 120, the signal passes through regulator 140 and pressure gauge 142 to a fifth input channel to bellows home valve 128 to provide a constant input signal thereto.

Because this valve is always left in A or "home" position after a use cycle, air from the fifth input channel at the beginning of a use cycle is deflected into the valve, and air from the first input channel exits via the second output channel to variable flow control device 130 having a check valve 134 and a variable restricted orifice 132 to regulate flow. Air exiting from flow control device 130 passes into lower chamber 136b of bellows drive cylinder 136 to force bellows drive piston 138 upwards to "home" position whereby the bellows is compressed.

From junction 122, the signal passes through regulator 144 to a first input channel of vacuum enable valve 146, a spring-return pressure piloted valve which can be purchased off the shelf, to provide a constant input thereto. From junction 122, also, the signal passes through regulator 150 and pressure gauge 152 to a fifth input channel to vacuum enable valve 146, to provide constant input thereto when the system is in run mode. Since vacuum enable valve 146 in default position passes a signal from fifth input channel out via fourth output channel, a constant signal input (so long as valve 146 is in default position) is provided to third input channel of pinch state valve 218, a detented pressure-piloted valve.

Pinch state valve 218, is left in B or "home" position after previous operation of the pump; as a result, the air from the third input channel exits via the second output channel to junction 236 to upper portion 238a of outlet pinch valve cylinder 238 and to lower portion 114b of inlet pinch valve cylinder 114 to force the outlet pinch valve piston 250 downward to "home" position to open the outlet valve and to force the inlet pinch valve piston 112 upward to "home" position to close the inlet valve.

From junction 116, the signal also passes via junction 118 to the first input channel to binary enable valve 164, a detented pressure-piloted valve, which, like all other valves in the system, is an off-the-shelf part. Also from junction 118, an air signal passes via junction 166, to, in turn, junctions 168, 170, 172, 174, 176, 178 and 180. Junctions 168, 170, 172, 174, and 176 pressurize respectively valves 162, 160, 158, 156, and 154 preferably on the front of the pump, which control, respectively, the following operational modes of the pump: withdraw, stop, oscillation type B, oscillation type A, and infusion. Thus, a constant input signal is provided to each of these toggle valves when the system is in run position.

In addition, junction 178 provides constant input to the counter 274, and junction 180 provides input into a first input channel of up relay valve 182, a spring-return pressure piloted valve and provides input to or receives output from "up amplifier" diaphragm 184, also an off-the-shelf unit.

Finally, from junction 166, a signal also passes to junction 316 and 318 to provide a first channel input to down relay valve 292, a spring-return pressure-piloted valve, and input to "down amplifier" diaphragm 290. From junction 316, a signal also passes through sensor regulator 310, pressure gauge 308, and junction 306. From junction 306, the two sensors, 268 and 188, are pressurized. Bottom sensor 268 is pressurized via a restricted orifice flow control device 286 which restricts the flow to junction 288 and from there to sensor 268 as well as down amplifier 290. The function of sensor 268, of course, is to sense when bellows drive piston 138 is at the bottom of its cycle and the bellows themselves extended, in order to activate upward piston movement and resulting bellows compression.

Similarly, top sensor 188 is pressurized from junction 306 via restricted orifice flow control device 190 and junction 186. Junction 186 also provides pressure to or allows back flow from sensor 188 to up amplifier 184. Top sensor 188 is designed to sense when the bellows drive piston 138 is at its uppermost position and the bellows compressed to activate downward piston movement and extension of the bellows.

Once priming of the pump is complete, thus, and the pump is to be used, the run/prime valve is toggled to run position which puts the pump in an initial home position as described above and which provides constant input as described above into certain valves of the system.

Activating the infusion valve 154, a momentary toggle valve, initially provides a signal to junctions 194. The infusion sequence will be described below in numbered steps.

I1. From junction 194, the signal passes to junction 270 and to OR gate 271 to activate the B pilot or "off" mode of top sensor enable valve 192, a detented pressure-piloted valve. (The word "mode" will frequently be used interchangeably with "pilot" hereafter,). Also from junction 270 and 276, the B or "off" mode of counter enable valve 272 is enabled. Finally, the B or "on" modes of force not home enable valve 214, and force home enable valve 230 are activated as well as the B mode of down relay valve 292, via junctions 278 and 284. The former two valves are detented pressure-piloted valves and the latter is a spring-return pressure-piloted valve.

I2. Also via junction 278, a signal passes to OR gate 280 to activate B or "off" mode of binary enable valve 164.

I3. Via junction 194 and 196, a signal is passed through OR gate 198 to junction 208 and passed to but restricted by fixed flow control device 210 having check valve 210a and restricted orifice 210b. Via junction 196, a signal passes to OR gate 220 and, via junction 222, activates the A or "delay" mode of the up timer select valve 212, a detented pressure-piloted valve. Also via junction 222, a signal activates the A or "delay" mode of the down timer select valve 224, also a detented pressure piloted valve. Via junction 208 an air signal is passed to a third input channel of force not home enable valve 214 which has already been set in B mode so that the signal passes through a fourth output channel to OR gate 216 and from there activates the A or "not home" mode of pinch state valve 218.

I4. Because third input channel of valve 218 has constant input when the pump is in run mode, and valve 146 is in default position, a signal is sent through valve 218 via its fourth output channel to junction 330. From junction 330 air passes to outlet pinch valve cylinder 238 in the lower portion 238a to force outlet pinch valve piston 250 upwards to pinch tubing 8b and close the outlet valve. Similarly, from junction 330, air passes through OR gate 108 to OR gate 110 to provide air pressure to inlet pinch valve cylinder 114, upper portion 114a thereof, to force inlet pinch valve piston 112 down to open the valve. Thus, the first steps of the infusion cycle change the valve positions from home position, closing the outlet valve and opening the inlet valve.

I5. In the meantime, the air signal exiting from junction 208 via flow control device 210 into the fourth output channel of up timer select valve 212 and out the third input channel because the valve is in A mode, to activate the B or "not home" mode of the bellows state valve 128. The constant input from the fifth input channel thus exits at the fourth output channel via variable flow control device 294 similar to variable flow control device 130 and from there to upper chamber 136a of bellows drive cylinder 136 to force bellows drive piston 138 down and extend the bellows.

I6. Thus, in this first portion of the infusion cycle, with the inlet valve open and the outlet valve closed, the bellows is extended to draw MTBE or other fluid from the reservoir into the bellows to fill the bellows to the preset position.

I7. As bellows drive piston moves down to the bottom of cylinder 138, it engages bottom sensor 268, so that air pressure originally exiting the sensor now forms a back flow to junction 288 and from there to diaphragm 290. When sufficient pressure accumulates in diaphragm 290, it activates the A pilot of down relay valve 292. Constant first channel input then exits through fourth channel output of the valve to junction 260. From junction 160, a signal passes to counter enable valve 272 but does not decrement the counter because the valve is in "off" mode.

I8. A signal also passes from junction 260 through second channel output of down timer select valve 224, exits first input channel and inputs again in the fifth input channel where it is diffused.

I9. A signal also passes from junction 260 to down delay timer 300 (consisting of spring return pressure-piloted valve 258, variable flow control device 264 and chamber 266) via junction 262 which directs the signal to first input channel of valve 258 as well as through variable flow control device 264 to accumulate in chamber 266. Only after sufficient pressure builds up in chamber 266, is the A mode of valve 258 activated, thus delaying the time when first channel input to valve 258 exits via second channel output to junction 228.

I10. From junction 228, a signal passes to the fifth input channel of force home enable valve 230 which is in "on" mode. The signal exits via the fourth output channel to OR gate 232 and 234 to activate the B or home mode of pinch state valve 218. As described before, since input to the third input channel is constant in run mode, the inlet and outlet pinch valve pistons 112 and 250 are thus forced to home position, up in the case of the inlet piston, to close the valve, and down in the case of the outlet piston, to open the valve.

I11. Also from junction 228, a signal passes to the fixed flow control device 226 through the down timer select valve 224 from the fourth output channel to the third input channel to activate the A or home mode of bellows state valve 128. In this mode, as described·before, constant input at the first input channel exits via the second input channel through flow control device 134 to bellows drive chamber 136b of bellows drive cylinder 136 to force bellows drive piston 138 to top position and compress the bellows. As a result, fluid in the bellows is passed out through the outlet valve through into a receiving unit, in the preferred embodiment, through a catheter into the gallbladder. Because the top sensor is disabled, the bellows remains in this position at the end of the infusion cycle, and the inlet and outlet valves remain in home position.

The preferred oscillation cycle, oscillation A, is activated by momentary toggle valve 156 which signals junction 313, OR gate 332, and the A or "on" mode of top sensor enable valve 192 via junction 334. From junction 334, the signal passes to junctions 336 and 338; from junction 336, it activates the A or "on" mode of counter enable valve 272, and from junction 338 the A or "off" modes of force home enable valve 214 and force not home enable valve 250. It will be described in numbered steps, below.

OA1. Also from junction 313, the air signal passes to junction 340 and 342. From junction 342, it activates the B, or "direct" mode of up timer select valve 212 and down timer select valve 224. From junction 340, the signal passes to junction 344 and OR gate 280 to activate the B or "off" mode of binary enable valve 164. From junction 344, it also passes to OR gate 234 to activate the B or "home" mode of pinch state valve 218. As explained previously, the constant input to this valve thus drives the inlet and outlet valves to home position, the former closed and the latter open. In addition, the valves for operation of the sensors have been enable and for delay timers have been disabled.

OA2. Constant input at the third channel to top sensor enable valve 192 thus exits through fourth output channel to junction 256. The signal passes to up delay timer 200 at junction 254. Up delay timer 200 consists of pressure-piloted spring-return valve 202, variable flow control device 204 containing a check valve 204a and a variable restricted orifice 204b as well as a chamber 206. From junction 254, a signal passes to the first channel of input to valve 202 as well as to the flow control device 204 and from there to chamber 206.

Thus, both valves controlling the timers are set in direct mode, so that no delay precedes change of bellows direction. Since there is no change of inlet and outlet valve state during oscillation A, no delay is necessary.

OA3. In addition from junction 256, input signal is provided to second channel input of up timer select valve 212, now in direct mode, and exits to activate the B or "not home" mode of bellows state valve 128. Constant input to that valve through the third channel exits through the fourth channel through flow control regulator 294 to fill upper chamber 136b of bellows drive cylinder 136 to force bellows drive piston 138 down to extend the bellows. Such bellows extension draws fluid from the available receptacle, here the gallbladder, in a preselected amount.

OA4. Pressure builds up at down sensor 268, causing backflow of air to junction 288 and to down amplifier diaphragm 290. When sufficient pressure builds up in the diaphragm, it activates the A mode of down relay valve 292, so that input signal at the first channel exits through the second channel to junction 260. At junction 260, the signal passes through the second channel out the third of down timer select valve 224 to activate the A or "home" mode of bellows state valve 128. From junction 260, input is also provided to down delay timer 300 at junction 254, and to the first channel of counter enable valve (now "on"), to exit at the second channel thereof, to decrement the counter 274. If the counter has not reached zero, the process continues as follows.

OA5. Bellows state valve 128, now in home position, allows constant input at its first channel to exit the second channel through flow regulator 134 to provide air pressure to lower chamber 136b of bellows drive cylinder 136 to drive bellows drive piston 138 upwards and compress the bellows, forcing the fluid just withdrawn into the bellows back into the gallbladder, to create turbulence in the gallbladder.

OA6. Once bellows drive piston reaches its uppermost position, top sensor 188 provides backflow to junction 186 and amplifier diaphragm 184. When diaphragm 184 expands sufficiently, it activates the A mode of spring-return pressure-piloted valve 182, the up relay valve, and constant input to the first channel provides a signal which exits the second channel to provide input to the third channel of top sensor enable valve 192. Because this valve is in A or "on" position, the signal exits through the fourth channel to junction 256 to repeat the cycle described above beginning with the fifth paragraph preceding this one.

OA7. This process is repeated until the counter reaches zero, indicating that the preset number of oscillations has been reached. When the counter reaches zero, the incoming constant input from junction 178 when the unit is in run mode, exits to junction 346 to OR gates 314 and 272 to activate the B or "off" mode of top sensor enable valve 192. Signal then exits through the second channel to OR gate 302 to activate the B mode of valve 304 and drive the valves home.

OA8. Also from junction 346, the signal passes to junction 344, where it enters the counter reset delay unit 348 consisting of spring-return pressure-piloted valve 350, variable flow control device 352 (having a check valve 352a and a variable restricted orifice 352b), and chamber 354. Input signal is provided to first channel of valve 350 as well as to flow control device 352 and chamber 354. When pressure reaches a sufficient level in chamber 354, the A mode of valve 350 is activated and the input at the first channel exits at the second channel through fixed flow control device 356 and returns to the counter to reset it and ready it for the next cycle.

To oscillate in B mode (from the gallbladder to the reservoir rather than just to the bellows), oscillation B valve 158, a momentary toggle valve, is toggled to send a signal to junction 370 which then activates the A or "on" mode of the binary enable valve 164, passes to OR gate 220 to activate, via junction 222, the A or "delay" mode of up timer select valve 212 and down timer select valve 224. In addition, a signal passes from junction 370 via OR gate 332 to junctions 334, 336 and 338 to activate the A or "on" modes of top sensor enable valve 192 and counter enable valve 272, plus the A or "off" modes of force not home enable valve 214 and force home enable valve 230. Because the top sensor enable valve 192 is left disabled initially, input backflow from the top sensor first passes through valve 192 at the second channel to OR gate 302 of binary section 382 to initially activate the B mode of valve 304. Oscillation B will be further described in numbered steps below.

OB1. Once the top sensor enable valve is activated, a signal passes through top sensor enable valve, now "on," to junction 256 to deadend in up timer select valve 212 via second channel input, first channel output, to fifth channel input.

OB2. From junction 256, a signal enters up delay timer 200 at junction 254, to pass to the first channel of valve 202 as well as variable flow control device 204 to chamber 206 to activate, after a delay, the A mode of valve 202.

OB3. Because binary section enable valve 164 is in A mode, constant input at the first channel provides an exit signal at the second channel to the third input channel of valve 304 which is in B mode. The signal exits via the second channel to junction 390 where it passes through OR gate 374 through flow restrictor 378 to activate the A mode of valve 380 (a momentary pressure-piloted valve). This momentarily allows flow through valve 380 from junction 376 to create pulsed flow through the valve, into OR gate 302 to activate the B mode of valve 304. In addition, from junction 390, a signal passes from OR gate 232 to OR gate 234 to activate the B or "home" mode of pinch state valve 218.

OB4. When sufficient pressure builds up in chamber 206 to activate the A mode of valve 202 of the up delay timer 200, the signal passes out through the second channel to junction 252, OR gate 198, junction 208, and into flow regulator 210. From junction 208, it passes to fifth channel of force not home enable valve 214 and deadends there.

OB5. From junction 252, a signal passes to the "binary section" 382 of the logic via junction 372 to OR gate 374 to junction 376 into restricted orifice 378 which in turn activates the A mode of momentary pressure-piloted valve 380. Also from junction 376, a signal passes to the second input to valve 380. From junction 372, a signal also passes to OR gate 392 of the binary section to junction 388 and flow restrictor 384. This activates the A mode of momentary pressure-piloted valve 386 to momentarily allow flow through the valve to activate the A mode of detented pressure-piloted valve 304.

OB6. As a result of the change in mode of valve 304, input from valve 164 to the third channel of 304 exits at the fourth channel to junction 394, OR gate 392, junction 388, flow restrictor 384, plus second channel input to valve 386. In addition, from junction 394, a signal passes to OR gate 216 to enable the A or not home mode of pinch state valve 218. In this mode, input to the third channel of pinch state valve 218 exits at the fourth channel thereof to signal the inlet and outlet valves to "not home" positions, i.e. inlet open and outlet closed, as described in detail above.

OB7. In the meantime, output signal from flow control device 210 passes through up timer select valve 212 to exit at channel five thereof and activate the B or "not home" mode of bellows state valve 128. As described before, this activates the bellows drive cylinder to lower and extend the bellows so as to withdraw liquid from the reservoir to the bellows.

OB8. When the bellows drive piston 138 reaches its lowest point, backflow from down sensor 268 reaches diaphragm 290 until sufficient pressure is reached for it to activate the A mode of down relay valve 292, at which point input to the valve exits at the second channel to junction 260. The signal then passes through the down timer select valve 224 which is in "delay" or A mode, and deadends by input to the fifth input channel thereof.

OB9. From junction 260, a signal passes to junction 262 to enter down delay timer 300 which, as described above, activates the A mode of valve 258, after a regulated delay. Finally, from junction 260, a signal passes to counter enable valve 272, which is in "on" mode, to exit at the second channel to decrement the counter 274. If the counter does not thereby reach zero, the cycle continues. A signal exits from valve 258 of down delay timer 300 to junction 228 and passes to the fifth channel input of force home enable valve 230 and deadends there because the valve is in "off" mode. A signal also passes through flow regulator 226 through down timer select valve 224 to activate the A or "home" mode of bellows state valve 128 to, as described above, compress the bellows and thereby reinfuse fluid to the main reservoir.

OB10. Backflow from top sensor 188 then activates the A mode of valve 182 to pass through counter enable valve 192 to junction 256 to activate up delay timer 200 and deadend in up timer select valve 212, all as previously described.

OB11. A signal passes from up delay timer valve 202 to deadend in force not home enable valve 272, to pass into flow control device 210, and to enter the binary section 382 at junction 372, all as described before.

OB12. If the valves are in home position, the B mode of valve 304 was last activated. Therefore, the A mode (which drives the valves to not home position) will be activated by the incoming signal to the binary section, and the entire system will pass through steps OB4 through OB10 numbered above, again.

OB13. If the valves are in not home position, the A mode of valve 304 was last activated, and as explained earlier, the signal passes from junction 372 to pulse valve 380, activating the B mode of valve 304. Signal passes through valve 302 back into valve 380 and also activates B or "home" mode of pinch state valve 218 to drive the valves home, all as described above.

OB14. At this point, steps OB7 through OB13 of the cycle are repeated.

OB15. If the counter decrements to zero, the steps described in OA8 and OA8 above are repeated and the valves returned to home, the bellows already being in home position.

To stop the device in an emergency situation, stop valve 160, a momentary toggle valve is toggled which sends a signal via OR gates 312, 314, and 272 to activate the B or "off" mode of counter enable valve 192 and disable the counter. Upon extension of bellows the bellows drive piston, the top sensor 188 is tripped. A signal passes through top sensor enable valve 192 to B mode of valve 304 via OR gate 302 to restore the home position of the pinch valves, if not already home, and halts the bellow drive.

To withdraw fluid from the gallbladder back to the fluid reservoir, withdraw valve 162, a momentary toggle valve is toggled. A signal passes to junction 358 to OR gates 312, 314, and 271, to disable the top sensor as in stop, described above.

In addition, a signal passes from junction 358 to junctions 360 and 362. From junction 360, the A or "on" mode of the vacuum enable valve 146 is activated. From junction 362, the signal passes to OR gate 110 to open the inlet valve as described above; thus, both the inlet and outlet valves are opened. In addition, the signal passes from junction 362 to activate the A or "not vented" mode of vacuum chamber vent valve 326, a momentary pressure-piloted valve. Constant input at the first channel exits from the second outlet of valve 146 to an off-the-shelf vacuum generator 364.

Vacuum is drawn at junction 366 from upper chamber 368a of diaphragm 368, from junction 324 measured by gauge 328. Diaphragm 368 acts as a safety mechanism during the withdraw cycle; if occlusion or the like occurs, it prevents positive pressure from building up in the vacuum system. (If a positive pressure is supplied, for whatever reason, to the inlet port of diaphragm 368, the diaphragm moves to occlude flow to its outlet, thus preventing positive pressures downstream. Only when a vacuum is supplied to its input will it allow flow.) Vacuum is thus drawn through the system to withdraw fluid from the gallbladder through the pump and to the reservoir.

To use the pump, the patient is first taken to radiology. Catheter 3 is inserted while the patient 23 is in radiology, and the pigtail is settled around the gallstones 5 in the gallbladder 4. Bile (23, see Fig. 1) is aspirated through the catheter, and imaging solution is then injected into the gallbladder until the gallbladder appears full. The amount of solution used is preferably recorded and approximately half the recorded amount used in the gallstone dissolution process. For example, if eight milliters are used to fill the gallbladder, approximately four milliliters will be infused into the gallbladder and then infused and aspirated repeatedly to create turbulence. In some circumstances, a smaller amount may be oscillated. Once the catheter has been placed in the gallbladder, it is left in place and used with the pump 2 for the gallstone dissolution process.

To use the pump, all the needed syringes 15 are filled with MTBE using the transfer cap 24 and empty syringes 10 are provided and attached to the system. MTBE is injected from input syringe 15 into the reservoir 16.

The compressed air source 100 is attached and the pressure regulated at regulator 102 if necessary. The prime toggle 106 on the pump should be on initially, driving the valves to home position. Syringe 10 is used for aspiration of the bile from the gallbladder and for aspiration of any air plus some MTBE to clear the system and is then sealed off. It can be used for hand infusion and oscillation if necessary but is normally left sealed off. Run/prime valve 106 is then toggled to run position A to initiate the system as described above.

The proper amount of MTBE (approximately four milliliters in this case) is then withdrawn from the main reservoir for infusion to the gallbladder. This is accomplished by hand selecting the appropriate volume with the volume control knobs 19 and 21 and by extension of the bellows with the valves in not home position. The valves are then placed in home position and the MTBE injected into the gallbladder by compression of the bellows to the home position. In the preferred embodiment, all of this is accomplished by pressing the infusion toggle 156 on the pump as described above.

The next step of the process is "oscillation" (repeated injection and aspiration of MTBE). The amount of liquid to be oscillated is first selected, the number of oscillations set on the counter 274 and an appropriate oscillation cycle chosen. Generally the entire amount infused will be oscillated, but a different amount can be selected; about 400 or so oscillations may be appropriate, and generally the oscillation A cycle is chosen because it is faster. Oscillation B, however, dilutes the solvent each cycle, which may be desireable on occasion, such as where the bellows is of small volume (i.e. as in Fig. 9), and further dilution is desireable.

Oscillation A is selected by toggling switch 156 on the front of the pump. With the valves in home position, the bellows repeatedly extends and compresses to withdraw the selected amount of fluid, usually one milliliter, from the gallbladder and infuse it back in for the selected number of cycles. This is accomplished by the air logic system 9 as explained above.

Oscillation B is selected by toggling switch 158 on the front of the pump. In oscillation B, the valves are set to not home position, and the bellows extends to withdraw fluid from the main syringe and the recompresses to return fluid to the main reservoir, having diluted the bellows fluid thus having been diluted with fresh solvent. The valves then switch to home position and the bellows compresses to inject the fluid into the gallbladder, then extends to withdraw it. This entire cycle is repeated until the preset number of cycles has been reached, all as described above.

Should the system need to be stopped, the stop button 160 is pressed, which returns everything to home position. Problems can then be handled, fresh MTBE added to the reservoir, or the like.

When oscillation is complete, the withdraw cycle is manually initiated, either because the entire procedure is complete or because a fresh aliquot of MTBE is desired. Withdraw toggle 162 on the front of the pump is hand activated and all fluid withdrawn from the system into the main reservoir, controlled by the air logic system as described above. Basically, the vacuum pump is activated, and main syringe 16 thereby caused to aspirate all the liquid in the system into the reservoir. Bile falls to the bottom, so that upon reinfusion, largely fresh MTBE is injected. If desired, an entirely new main syringe can be inserted and filled before the next infusion. Finally, if the entire dissolution process is completed (which is determined by reinjecting imaging solution into the gallbladder via syringe 10), the withdraw cycle is the last stage of the process.

Finally, the catheter is removed from the pump and from the patient, now happily free of gallstones dissolved by the above process.

**Claims**

1. In a syringe having a barrel and a plunger having a piston slideably and sealably received within the bore of the barrel, the improvement which comprises a piston of reduced diameter relative to the bore of the barrel for part of its length whereby a tendency of the piston to become immobilized relative to the barrel is minimized.

2. In a syringe also having a plunger terminating in a cap external to the barrel at the proximal end and a tip at the distal end, the improvement of claim 1 which comprises a piston having said reduced diameter proximal to the tip.

3. In a glass syringe, the improvement of claim 2 wherein more than about three-fourths the length of the piston is of said reduced diameter.

4. In a glass syringe, the improvement of claim 4 and wherein said diameter is reduced to about 96 percent of the diameter of the tip.

5. Apparatus for transferring fluid comprising: a fluid source;

transfer means for transferring fluid to or from the fluid source and a fluid receiver;

first means for closing off fluid flow from the source to the transfer means,

second means for closing off fluid flow from the transfer means to the receiver;

control means for independently controlling the transfer means and the first and the second closing means so that a) fluid can be transferred from the fluid source to the transfer means, from the transfer means to the fluid receiver, and back again, at the user's selection.

6. Apparatus according to claim 5 and wherein the control means functions so that b) fluid can also be repeatedly transferred directly from the transfer means to the fluid receiver and back again, at the user's selection.

7. Apparatus according to claim 6 and wherein the control means functions so that c) fluid can also be repeatedly transferred directly from the fluid source to the transfer means and back again, at the user's selection.

8. Apparatus according to claim 7 and wherein the control means functions so that step b) can be repeatedly alternated with step c) at the user's selection.

9. Apparatus according to claim 5 and wherein the transfer means is a positive displacement source.

10. Apparatus according to claim 9 and wherein the positive displacement source is a disposable unit.

11. Apparatus according to claim 9 and wherein the positive displacement source may contain two phases of a liquid and differentially delivers a phase to the receiver or the source.

12. Apparatus according to claim 9 and wherein the positive displacement source is a bellows.

13. Apparatus according to claim 12 and including a connector between the bellows and the fluid source disposed at the bottom of the bellows so that the lower phase of two phases of liquid in the bellows is differentially delivered to the fluid source.

14. Apparatus according to claim 5 and wherein the first and second closing means are pinch valves.

15. Apparatus according to claim 5 and wherein the control means is a fluid logic system.

16. Apparatus according to claim 5 and wherein the fluid logic system is a pneumatic logic system.

17. Apparatus according to claim 5 and further including means for adjusting the volume of fluid transferred by the transferring means.

18. Apparatus according to claim 5 and wherein the fluid source is graduated to measure the amount of fluid transferred to and from the fluid receiver.

19. Apparatus according to claim 5 and wherein the fluid source may contain liquid in two phases.

20. Apparatus according to claim 19 and wherein the fluid source differentially delivers one phase of the two phases of liquid to the transferring means.

21. Apparatus according to claim 5 further including a vacuum generator, and a vacuum chamber surrounding the fluid source, and wherein the fluid source is a syringe so that air leakage into the apparatus through the syringe is minimized by use of the vacuum to withdraw the plunger of the syringe.

22. Apparatus according to claim 5 and wherein the fluid is methyl tertiary-butyl ether, the fluid receiver is the gallbladder, the transferring means is a bellows, and the control means is a pneumatic logic system.

23. Pump apparatus comprising:

a fluid source;

means for transferring fluid to or from the fluid source and a fluid receiver;

pneumatic logic control means to control the transfer of fluid from the fluid source to or from the fluid receiver.

24. Pump apparatus according to claim 23 and wherein the transferring means is a positive displacement means.

25. Pump apparatus according to claim 25 further including:

first means for closing off fluid flow from the source to the transfer means;

second means for closing off fluid flow from the transfer means to the receiver;

and wherein the pneumatic logic control means independently controls the transfer means and the first and the second closing means so that a) fluid can be transferred from the fluid source to the transfer means, from the transfer means to the fluid receiver and back again, at the user's selection.

26. Pump apparatus according to claim 25 and wherein the control means functions so that b) fluid can be repeatedly transferred directly from the transfer means to the fluid receiver and back again, at the user's selection.

27. Pump apparatus according to claim 26 and wherein the control means functions so that c) fluid can be repeated transferred directly from the fluid source to the transfer means and back again, at the user's selection.

28. Pump apparatus according to claim 27 and wherein the control means functions so that step b) can be repeated alternately with step c) at the user's selection.

29. Pump apparatus according to claim 24 and wherein the positive displacement means is a disposable unit.

30. Pump apparatus according to claim 24 and wherein the positive displacement means may contain two phases of a liquid and differentially delivers one phase to the receiver or the source.

31. Pump apparatus according to claim 24 and wherein the positive displacement means is a bellows.

32. Pump apparatus according to claim 31 and including a connector between the bellows and the fluid source disposed at the bottom of the bellows so that the lower phase of two phases of liquid in the bellows is differentially delivered to the fluid source.

33. Pump apparatus according to claim 25 and wherein the first and second closing means are pinch valves.

34. Pump apparatus according to claim 23 and further including means for adjusting the volume of fluid transferred by the transferring means.

35. Pump apparatus according to claim 23 and wherein the fluid source is graduated to measure the amount of fluid transferred to and from the fluid receiver.

36. Pump apparatus according to claim 35 and wherein the fluid source may contain liquid in two phases.

37. Pump apparatus according to claim 36 and wherein the fluid source differentially delivers one phase of the two phases of liquid to the transferring means.

38. Pump apparatus according to claim 23 further including a vacuum generator and a vacuum chamber surrounding the fluid source and wherein the fluid source is a syringe so that air leakage into the apparatus is minimized by use of the vacuum to withdraw the plunger of the syringe.

39. Pump apparatus according to claim 25 and wherein the fluid is methyl tertiary-butyl ether, the fluid receiver is the gallbladder, the transferring means is a bellows, and the fluid source is an inverted syringe.

40. A method for dissolving solidified masses in a localized area of the body, in vivo, by infusing a given volume of fluid into said localized area and oscillating a smaller volume to and from said localized area to dissolve said masses therein.

41. A method according to claim 40 including the step of withdrawing said fluid from the localized area.

42. A method according to claim 40 and wherein the masses are gallstones.

43. A method according to claim 40 and wherein the localized area is the gallbladder.

44. A method according to claim 40 and wherein the fluid includes monooctanoin or methyl tertiary-butyl ether.

45. A method according to claim 44 and wherein the fluid consists essentially of methyl tertiary-butyl ether.

46. A cap for a fluid container comprising a cap unit and tubing, the tubing disposed to extend through the cap unit into the container at one end and terminating in a luer seal at the other to facilitate withdrawal of liquid from the bottle by syringe.

47. A cap according to claim 46 and wherein the cap includes means for closing off liquid in the container from the environment.

48. A cap according to claim 47 and wherein the closing means is a sliding clamp.

49. Apparatus for withdrawing the plunger of a syringe with minimized air leakage including a syringe, a vacuum generator, and a vacuum chamber surrounding the syringe.

VACUUM
EXHAUST

FIG. 1

EP 0 358 429 A2

FIG. 2

*FIG. 3*

*FIG. 4*

65

63

## FIG. 8

50

60

58

11

## FIG. 7

7

60

81

8

80

## FIG. 15

3

50

51

52

## FIG. 6

## FIG. 5

FIG. 9

FIG. 10

FIG. 11

FIG. 12

FIG. 13

FIG. 14

**FIG. 16**

FIG. 17

FIG. 18